# FASCICULE DE BREVET EUROPEEN

(11) **EP 2 654 762 B1**
(45) Date de publication et mention de la délivrance du brevet: **26.09.2018**
(21) Numéro de dépôt: 11804695.2
(22) Date de dépôt: 22.12.2011
(51) Int. Cl.: A61K 36/185, A61K 8/97, A61Q 19/00, A61P 9/00, A61P 17/00, A61K 8/60, A61Q 19/06, A61Q 19/08

(54) **EXTRAIT DE PARTIES AERIENNES DE GYNANDROPSIS GYNANDRA OU CLEOME GYNANDRA ET COMPOSITIONS COSMETIQUES, DERMATOLOGIQUES OU PHARMACEUTIQUES LE COMPRENANT**
EXTRAKTE AUS DEN OBERIRDISCHE TEILE DES GYNANDROPSIS GYNANDRA UND KOSMETISCHE, DERMATOLOGISCHE SOWIE PHARMAZEUTISCHE ZUSAMMENSETZUNGEN DIE DIESE EXTRAKTE BEINHALTEN
EXTRACT OF THE AERIAL PARTS OF GYNANDROPSIS GYNANDRA AND COSMETIC, DERMATOLIGIC AND PHARMACEUTIC COMPOSITIONS COMPRISING THE EXTRACT

(30) Priorité: 22.12.2010 FR 1061051
(43) Date de publication de la demande: 30.10.2013
(73) Titulaire: Laboratoires Expanscience, 92048 Paris La Défense Cedex (FR)
(72) Inventeur: MSIKA, Philippe, F-78000 Versailles (FR); SAUNOIS, Alex, F-28210 Nogent-Le-Roi (FR); BAUDOUIN, Caroline, F-78120 Rambouillet (FR); LECLERE-BIENFAIT, Sophie, F-28100 Dreux (FR); DEBROCK, Sebastien, F-28130 Saint-Martin De Nigelles (FR)
(74) Mandataire: Regimbeau
(86) Numéro de dépôt international: PCT/EP2011/073838
(87) Numéro de publication internationale: WO 2012/085230

(56) Documents cités:
- EP-A1- 1 352 640
- FR-A1- 2 945 943
- "Philippine medicinal plants: Apoi-apoian", , 1 août 2010 (2010-08-01), XP002651621, Extrait de l'Internet: URL:http://www.stuartxchange.org/Apoi-apoi an.html [extrait le 2011-07-19]
- HOWARD ET AL: "Are there Customary Rights to Plants? An Inquiry among the Baganda (Uganda), with Special Attention to Gender", WORLD DEVELOPMENT, PERGAMON, vol. 35, no. 9, 31 août 2007 (2007-08-31), pages 1542-1563, XP022227162, ISSN: 0305-750X, DOI: DOI:10.1016/J.WORLDDEV.2006.05.021
- VIJAYAKUMAR J ET AL: "Antibacterial activity of extracts of cleome Gynandra L", GEOBIOS, UNIVERSITY OF JODHPUR, JODHPUR, IN, vol. 32, no. 1, 1 janvier 2005 (2005-01-01), pages 8-10, XP009150403, ISSN: 0251-1223
- GHOGARE U R ET AL: "Antinociceptive activity of Gynandropsis gynandra leaves", NATURAL PRODUCT RESEARCH, TAYLOR & FRANCIS, LONDON, vol. 23, no. 4, 1 janvier 2009 (2009-01-01), pages 327-333, XP009150404, ISSN: 1478-6427
- NARENDHIRAKANNAN ET AL: "Anti-inflammatory and lysosomal stability actions of Cleome gynandra L. studied in adjuvant induced arthritic rats", FOOD AND CHEMICAL TOXICOLOGY, PERGAMON, GB, vol. 45, no. 6, 3 avril 2007 (2007-04-03), pages 1001-1012, XP022015672, ISSN: 0278-6915, DOI: DOI:10.1016/J.FCT.2006.12.009
- AJAIYEOBA: "Phytochemical and antimicrobial studies of Gynandropsis gynandrauchholzia coriaceae extracts", AFR. J. BIOMED. RES., vol. 3, 2000, pages 161-165, XP002651622,
- COOK J A ET AL: "USE OF THE TROLOX ASSAY TO ESTIMATE THE ANTIOXIDANT CONTENT OF SEVENTEEN EDIBLE WILD PLANTS OF NIGER", LIFE SCIENCES, PERGAMON PRESS, OXFORD, GB, vol. 63, no. 2, 5 juin 1998 (1998-06-05), pages 105-110, XP009057822, ISSN: 0024-3205, DOI: DOI:10.1016/S0024-3205(98)00245-8
- LWANDE W ET AL: "Gynandropsis gynandra essential oil and its constituents as tick (Rhipicephalus appendiculatus) repellents", PHYTOCHEMISTRY, PERGAMON PRESS, GB, vol. 50, no. 3, 10 février 1998 (1998-02-10), pages 401-405, XP004290868, ISSN: 0031-9422, DOI: DOI:10.1016/S0031-9422(98)00507-X
- BALA A ET AL: "Evaluation of anticancer activity of Cleome gynandra on Ehrlich's Ascites Carcinoma treated mice", JOURNAL OF ETHNOPHARMACOLOGY, ELSEVIER SCIENTIFIC PUBLISHERS LTD, IE, vol. 129, no. 1, 4 mai 2010 (2010-05-04), pages 131-134, XP027013341, ISSN: 0378-8741 [extrait le 2010-03-20]
- SHARAF M ET AL: "Exudate flavonoids from aerial parts of four Cleome species", BIOCHEMICAL SYSTEMATICS AND ECOLOGY, PERGAMON PRESS, GB, vol. 20, no. 5, 1 juillet 1992 (1992-07-01), pages 443-448, XP025667812, ISSN: 0305-1978, DOI: 10.1016/0305-1978(92)90084-Q [extrait le 1992-07-01]

## Description

L'invention se rapporte à une composition, de préférence cosmétique, dermatologique ou pharmaceutique, comprenant un extrait de parties aériennes de *Gynandropsis gynandra,* plus avantageusement de feuilles, et avantageusement un excipient approprié.

Avantageusement, l'extrait de parties aériennes de *Gynandropsis gynandra,* dans la composition, a une teneur comprise entre 0,01% et 10% en poids par rapport au poids total de la composition.

L'invention a également pour objet un procédé d'extraction d'un extrait de parties aériennes de *Gynandropsis gynandra* et plus avantageusement de feuilles, ainsi que l'extrait susceptible d'être obtenu par ledit procédé.

L'invention concerne également une telle composition ou un tel extrait pour son utilisation dans la prévention ou le traitement des troubles ou pathologies de la peau, des muqueuses ou des phanères, pour son utilisation dans la prévention ou le traitement des troubles vasculaires et/ou des problèmes liés à l'hyperséborrhée, ou pour son utilisation en tant que produit anti-acnéique, anti-âge, cicatrisant, hydratant, amincissant et/ou anticellulite, anti-allergique et pro-pigmentant. L'invention concerne enfin un procédé de soin cosmétique de la peau, des phanères ou des muqueuses, en vue d'améliorer leur état ou leur aspect, consistant à administrer une telle composition ou un tel extrait.

*Gynandropsis gynandra* est une plante de la famille des Capparacées. Ses noms botaniques sont les suivants : *Gynandropsis gynandra* (L.) Briq ou *Gyandropsis pentaphylla* ou *Cleome gynandra.* Elle est également désignée sous divers noms vernaculaires, qui varient en fonction des zones géographiques. On pourra citer :
▪ « wouin wouin » en Jula (sud ouest du Burkina) et Kyenebdo en Moré (centre du Burkina Faso) et nombreuses dénominations différentes dans les différents pays d'Afrique ;
▪ Cleome, Gynandro, Mouzambe à fleurs blanches en français ;
▪ Cat's whiskers ou spider flower en anglais ; et
▪ Senfkapper, benzoinbaun en allemand.

C'est une petite plante herbacée annuelle, plante cultivée de 15 à 30 cm de hauteur, pouvant atteindre 1,5 m de hauteur sous sa forme sauvage, à petites feuilles, tiges rondes, parfois rougeâtres. La floraison est constituée de petites fleurs bleues à violettes pâles. Les fruits sont des gousses allongées de 4 à 6 cm de long, qui contiennent de nombreuses graines, minuscules, noirâtres.

Il a été décrit que les feuilles et tiges contiennent les composés suivants :
▪ Isothiocyanate (cléomine),
▪ Noyaux stéroliques (lupéol, campestérol, épi-lupéol),
▪ Vitamine C (127 - 484 mg/100g),
▪ β-carotène (6,7 - 18,9 mg/100g),
▪ Carbohydrates (4,4 - 6,4 %),
▪ Protéines (3,1 - 7,7%),
▪ Composés phénoliques (520 - 910 mg/100g),
▪ Calcium (213 - 434 mg/100g), et
▪ Magnésium (86 mg/100g).

La présence d'alcaloïdes est également mentionnée par certains auteurs, mais pas par d'autres.

Il a été décrit que les feuilles et tiges contiennent les composés suivants :
▪ 29,5% de protéines
▪ 28% lipides (59% acide linoléique et environ 20% d'acide oléique)
▪ Stérols
▪ Composés phénoliques (kaempférol, lutéoline).

Les parties aériennes (tige et feuilles) sont traditionnellement utilisées pour une administration par voie orale. Compte-tenu de sa réputation de plante à haute teneur en vitamines et micro-nutriments, elle est le plus souvent utilisée comme plante alimentaire, aussi bien chez l'adulte que chez l'enfant et fortement recommandée chez la femme enceinte en tant que condiment pour les sauces et également en tant que légumes en soupes le plus souvent.

Les parties aériennes et les tourteaux obtenus après extraction de l'huile des graines peuvent être utilisés comme fourrage pour le bétail.

Par ailleurs, des préparations de feuilles, de tiges et de graines, auraient des vertus insecticides, vermifuges, anti-parasitaires et anti-microbiennes et anti-oxydantes lorsqu'elles sont consommées par voie orale. Le jus de feuilles serait utilisé en lavages oculaires. Les graines, consommées par voie orale, présenteraient des vertus anti-vomitives.

Dans différentes médecines traditionnelles (en particulier en Afrique ou en Inde), la plante est utilisée par voie orale essentiellement, pour le traitement des douleurs (maux de tête, douleurs liées à l'accouchement, estomac), des morsures de scorpion. Les feuilles peuvent être appliquées directement sur des plaies purulentes pour prévenir la formation de pus.

Des études récentes pharmacologiques ont conforté certains de ces usages traditionnels : activité analgésique chez la souris par injection intra-péritonéale d'extraits de la plante (U.R. Ghogare et Coll., Natural Product Research, 23 (4) : 327-333 (2009)) ; effet anti-arthrite chez le rat par voie orale (R.T. Narendhirakannan et Coll., Molecular and Cellular Biochemistry, 276 : 71-80 (2005)) ; effet bénéfique anti-cholestérol par voie orale (T. Johns, Journal of Ethnopharmocology, 66 : 1-10 (1999)).

Le document *Philippine medicinal plants : Apoi-apoian* est une étude botanique sur la plante *Gynandropsis Gynandra*, qui peut être utilisée pour le traitement de diverses maladies de peau.

Le document Narendhirakkannan *et al.* décrit un extrait de feuilles sèches de Gynandropsis Gynandra obtenu avec de l'éthanol à 95%.

Le document FR 2 945 943 concerne l'utilisation d'un extrait végétal riche en polyphénols comme agent actif cosmétique. Ce document porte plus particulièrement sur l'utilisation d'un extrait de rose présentant naturellement une teneur élevée en anthocyanes, comme agent anti-oxydant.

Par ailleurs, la demande américaine US 2004/0028643A1 décrit le screening de nombreuses plantes, pour leur utilisation dans des compositions anti-âge. Les résultats donnés dans les exemples de cette demande américaine laissent apparaître que l'extrait de plante de *Cleome gynandra* (l'extrait méthanolique d'une partie non définie ou de la totalité de la plante) ne montre pas de résultats satisfaisants. En particulier, suite au test activité anti-DPPH, l'extrait n'a pas été retenu comme présentant une activité significative.

Au contraire, les inventeurs ont découvert que les extraits de parties aériennes (feuilles, tiges, fleurs, graines) de *Gynandropsis gynandra,* et avantageusement des extraits de feuilles, présentaient des propriétés cosmétiques et dermatologiques jamais décrites jusqu'à présent. Les exemples qui suivent montrent que l'extrait selon l'invention présente une activité anti-DPPH significative.

C'est la première fois que des extraits de parties aériennes de *Gynandropsis gynandra* sont utilisés en tant que tels, pour leurs propriétés spécifiques.

L'invention a pour objet une composition comprenant à titre de principe actif un extrait de parties aériennes de *Gynandropsis gynandra* (ci-après dénommé extrait selon l'invention), avantageusement d'un extrait de feuilles de *Gynandropsis gynandra,* caractérisée en ce que l'extrait comprend au moins 3 % en poids de polyphénols, exprimé en équivalent d'acide gallique par rapport au poids de l'extrait sec.

Avantageusement, les polyphénols sont des flavonoïdes. Selon ce caractère particulier, l'extrait comprend au moins 1 % en poids de flavonoïdes, avantageusement au moins 3 % en poids, exprimé en équivalent de rutine par rapport au poids de l'extrait sec.

L'extrait de *Gynandropsis gynandra*, selon l'invention, est éventuellement en association avec un excipient approprié dans la composition.

La composition est avantageusement une composition cosmétique, dermatologique, ou pharmaceutique.

La composition est avantageusement destinée à être appliquée par voie topique externe sur la peau, les phanères et/ou les muqueuses, en particulier les peaux, les phanères et/ou les muqueuses sensibles ou agressées par l'environnement, par exemple par les UV ou la pollution.

L'extrait est avantageusement un extrait de feuilles de *Gynandropsis gynandra.*

L'extrait selon l'invention se caractérise par sa teneur en polyphénols (dosage de Folin-Ciocalteu) qui est d'au moins 3% en poids exprimé en équivalent d'acide gallique par rapport au poids de l'extrait sec.

Parmi les polyphénols présents dans cet extrait, les flavonoïdes sont particulièrement intéressants. Ainsi, dans une variante avantageuse de l'invention, l'extrait selon l'invention peut se caractériser par sa teneur en flavonoïdes (dosage au chlorure d'aluminium), qui est d'au moins 1% en poids de flavonoïdes exprimé en équivalent rutine par rapport au poids de l'extrait sec. Ces flavonoïdes sont préférentiellement constitués majoritairement de rutine et de ses dérivés.

La rutine est un composé de formule (I) :

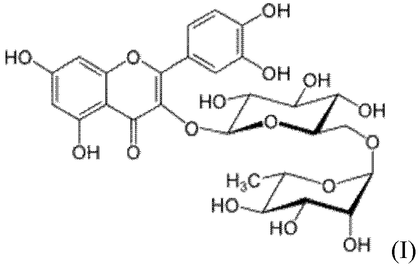

Les dérivés de la rutine sont avantageusement les composés dans lesquels on retrouve le résidu flavonique de la rutine, résidu de formule (II) :

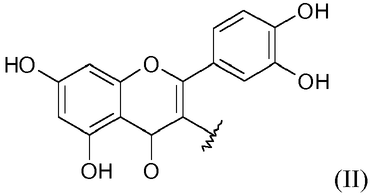

Dans les flavonoïdes contenus dans l'extrait selon l'invention, on retrouve avantageusement la rutine et ses dérivés dans une proportion, en poids exprimée en équivalent rutine par rapport à la teneur en poids total de flavonoïdes, d'au moins 50%, avantageusement au moins 60%, plus avantageusement au moins 70%, encore plus avantageusement au moins 80%.

Selon une variante avantageuse de l'invention, l'extrait contient de 0% à 80%, avantageusement de 10 à 80%, plus avantageusement de 30 à 70% de sucres, les % étant exprimés en poids par rapport au poids de l'extrait sec.

L'extrait selon l'invention contient avantageusement 0 à 50% en poids, plus avantageusement 0 à 20% en poids, encore plus avantageusement 0 à 10% en poids de lipides de *Gynandropsis gynandra*; les % étant exprimés en poids par rapport au poids total de l'extrait sec.

L'extrait selon l'invention contient avantageusement 0 à 60% en poids, plus avantageusement 0,5 à 30% en poids, encore plus avantageusement 0,5 à 10% en poids de protéines de *Gynandropsis gynandra*; les % étant exprimés en poids par rapport au poids total de l'extrait sec (dosage de Bradford).

Cet extrait est susceptible d'être obtenu par extraction solide-liquide des parties aériennes, fraîches ou sèches, de *Gynandropsis gynandra* dans un solvant aqueux et glycolique et/ou glycérolique. Les parties aériennes sont avantageusement les feuilles.

Le solvant est choisi parmi les mélanges binaires d'eau avec le glycérol, un glycol comme le propanediol, ou leurs mélanges, dans des proportions comprises entre 10% et 50% d'eau par rapport aux autres solvants.

Majoritairement, on utilise des mélanges binaires de solvants du type eau et un solvant choisi parmi le glycérol ou le propanediol.

Plus particulièrement, on introduira entre 0,1 et 50% en poids (en équivalent matière sèche) de parties de plante souhaitée dans le solvant d'extraction, et préférentiellement entre 1 et 10% en poids, avantageusement 5% en poids (les % sont exprimés en poids de la matière sèche par rapport au poids total mis en oeuvre). La partie sèche de *Gynandropsis gynandra* peut être les feuilles, les tiges, les fleurs, les graines, seules ou associées, et préférentiellement les feuilles.

En présence de glycérol, on choisira une proportion comprise entre 0 et 100% de glycérol dans l'eau, préférentiellement entre 30 et 80%, et avantageusement 80% (les % sont exprimés en poids de glycérol par rapport au poids total eau+glycérol).

En présence de glycol et plus particulièrement de propanediol, on choisira une proportion comprise entre 0 et 100% de propanediol dans l'eau, préférentiellement entre 10 et 80%, et avantageusement 60% (les % sont exprimés en poids de propanediol par rapport au poids total eau+propanediol).

La température d'extraction est avantageusement comprise entre 4°C et 100°C, et préférentiellement entre 10°C et 60°C, et plus particulièrement entre 15°C et 30°C.

La durée d'extraction varie avantageusement de 30 minutes à 4 heures, et préférentiellement de 30 minutes à 2 heures, et plus avantageusement elle est d'environ 1 heure.

A l'issue de l'extraction, la matière sèche résiduelle est avantageusement séparée de la phase liquide, par exemple par filtration, décantation ou centrifugation. La phase liquide ainsi obtenue peut être filtrée à l'aide de filtres de porosité adaptée afin d'obtenir une solution limpide.

Ces premières étapes de séparation peuvent être suivies d'étapes de purification, par exemple par ultrafiltration et/ou nanofiltration, permettant de concentrer les molécules d'intérêt potentielles au dépend d'autres.

L'extrait obtenu pourra se présenter sous forme liquide mais également pourra être séché selon les méthodes connues de l'Homme de l'art, l'atomisation ou la lyophilisation par exemple avec, ou sans support telle la maltodextrine.

L'invention a également pour objet un procédé de préparation d'un extrait de parties aériennes de *Gynandropsis gynandra* comprenant les étapes successives suivantes :
(a) dispersion en phase liquide dans un solvant adapté de parties aériennes de *Gynandropsis gynandra,* et avantageusement des feuilles,
(b) soumission du mélange obtenu suite à l'étape (a) à une extraction dans un solvant aqueux et et/ou glycolique et/ou glycérolique;
(c) centrifugation et/ou filtration de l'extrait obtenu suite à l'étape (b) ;
(d) le cas échéant, ultrafiltration et/ou diafiltration et/ou nanofiltration de l'extrait obtenu suite à l'étape (c) ;
(e) suite à l'étape (c) ou (d), récupération de l'extrait de parties aériennes de *Gynandropsis gynandra ;*
(f) séchage éventuel de l'extrait obtenu à l'étape (e) sur un support ou non.

Lors de l'étape (a), on utilise avantageusement les feuilles dans les proportions suivantes : entre 0,1 et 50% de matière sèche de feuilles, préférentiellement entre 5 et 20%, et avantageusement 5%, les pourcentages étant exprimés en poids de la matière sèche par rapport au poids total mis en oeuvre.

L'étape (b) se fait avantageusement sous agitation. Aucune enzyme n'a à être ajoutée.

Lors de l'étape (c), on utilise avantageusement les solvants choisis parmi les mélanges binaires d'eau avec le glycérol, un glycol comme le propanediol, ou leurs mélanges dans une proportion avantageusement comprise entre 30 et 90% de ces solvants dans l'eau et plus avantageusement entre 50 et 80% (les % sont exprimés en poids du solvant par rapport au poids total solvant+eau).

L'extrait est avantageusement utilisé en tant qu'agent actif dans une composition telle qu'une composition cosmétique, dermatologique ou pharmaceutique, qui peut comprendre un ou plusieurs excipients appropriés. La composition peut en outre comprendre au moins un autre composé actif en plus de l'extrait de parties aériennes de *Gynandropsis gynandra.* Cet autre composé peut être choisi parmi tous les composés et leurs équivalents fonctionnels, énoncés ci-dessous.

Cet autre composé peut être en particulier choisi parmi des actifs classiquement utilisés en dermatologie, pharmacologie ou en cosmétique et connus de l'homme de l'art tels que les émollients, les actifs hydratants, les kératorégulateurs, les kératolytiques, les agents cicatrisant et/ou restructurant de la barrière cutanée, les agonistes PPAR, RXR ou LXR, les agents sébo-régulateurs, les agents anti-irritants et/ou anti-inflammatoires et/ou apaisants, les agents anti-oxydants, les agents anti-âge, les agents dépigmentants ou hypopigmentants, les agents pigmentants, les agents lipolytiques ou inhibiteurs de la lipogénèse ou encore les agents anti-cellulite ou amincissants, les filtres et écrans solaires minéraux ou organiques, les composés antifongiques, les conservateurs, les agents anti-bactériens, les pré et probiotiques, les antibiotiques, les immunomodulateurs.

Plus particulièrement, les agents cicatrisants et/ou restructurants de la barrière cutanée pouvant être utilisés en association sont avantageusement le panthénol (vitamine B5), l'arabinogalactane, l'oxyde de zinc, les céramides, le cholestérol, le squalane et les phospholipides.

Les agents sébo-régulateurs pouvant être utilisés en association sont avantageusement choisis dans le groupe constitué par les inhibiteurs de 5-alpha réductase. Le zinc (et les dérivés du zinc tel que ses sels gluconate, salicylate et acide pyroglutamique) et la spironolactone, présentent aussi une activité sébo-suppresseur. D'autres séborégulateurs d'origine lipidique agissant sur la qualité du sébum, comme l'acide linoléique présentent un intérêt.

L'agent anti-inflammatoire et/ou anti-irritant et/ou apaisant peut être l' arabinogalactane.

Les actifs protecteurs solaires pouvant être utilisés en association sont avantageusement des filtres ou écrans solaires UVB et/ou UVA ; tels les écrans ou filtres minéraux et/ou organiques connus de l'homme du métier qui adaptera leur choix et leurs concentrations en fonction du degré de protection recherché.

Les conservateurs pouvant être utilisés en association sont par exemple ceux généralement utilisés en cosmétique, les molécules à activité anti-bactérienne (pseudo-conservateurs) tels que les dérivés capryliques comme par exemple le capryloyl glycine et le glycéryl caprylate ; l'hexanediol, le sodium levulinate, et les dérivés de zinc et de cuivre (gluconate et PCA).

Parmi les actifs recommandés en association avec l'extrait selon l'invention, on peut citer les extraits végétaux, en particulier :
- les huiles végétales telles que les huiles de Soja et/ou l'huile de Colza, l'huile d'avocat (WO2004/012496, WO2004/012752, WO2004/016106, WO2007/057439), l'huile de Lupin, avantageusement l'huile de Lupin blanc doux (WO 98/47479), ou un mélange de ces huiles ;
- l'oléodistillat ou les concentrats d'huile végétale ou animale, notamment de tournesol, plus avantageusement des concentrats de Tournesol linoléiques, tels que l'huile de tournesol concentrée en insaponifiables (Soline®) (cf. la demande internationale WO 01/21150) commercialisée par les Laboratoires Expanscience, les huiles concentrées en insaponifiable du type huile d'avocat, de colza, de maïs ou de palme, utiles notamment pour leur activité hydratante et/ou émolliente, cicatrisante et/ou restructurante de la barrière cutanée, anti-inflammatoire et/ou anti-irritante et/ou apaisante ;
- les insaponifiables de végétaux ou d'huile végétale, avantageusement des furanes d'avocat (Avocadofurane®), pouvant être obtenus par le procédé décrit dans la demande internationale WO 01/21605, les insaponifiables d'Avocat et/ou de Soja, plus particulièrement un mélange d'insaponifiables d'Avocat furaniques et d'insaponifiables de Soja, avantageusement dans un rapport respectif d'environ 1/3-2/3 (tel que Piasclédine®), les insaponifiables de soja (tels qu'obtenus selon le procédé décrit dans la demande internationale WO 01/51596), les insaponifiables stéroliques (typiquement des insaponifiables dont la teneur en stérols, en méthylstérols et en alcools triterpèniques est comprise entre 20 et 95 % en poids, de préférence 45-65 % en poids, par rapport au poids total de l'insaponifiable), les phytostérols, les esters de stérols et les dérivés vitaminiques, utiles notamment pour leur activité cicatrisante et/ou restructurante de la barrière cutanée, anti-âge, anti-inflammatoire ;
- les peptides ou complexes d'acides aminés végétaux, en particulier les peptides d'avocat (tel que ceux décrits dans la demande internationale WO2005/105123), les peptides de lupin (tels que ceux obtenus selon le procédé décrit dans la demande WO2005/102259), les peptides de quinoa (tel que ceux décrits dans la demande internationale WO2008/080974), les peptides de Maca tel que ceux décrits dans la demande internationale WO2004/112742), les peptides de soja fermenté ou non, les peptides de riz (tel que ceux décrits dans la demande internationale WO 2008/009709), utiles notamment pour leur activité hydratante et/ou émolliente (avocat), kératorégulatrice (lupin, quinoa), cicatrisante et/ou restructurante de la barrière cutanée (maca, quinoa, soja), anti-inflammatoire et/ou anti-irritante et/ou apaisante (lupin, quinoa), antioxydante (avocat), anti-âge (lupin, maca), pigmentante (riz), les peptides de Schizandra (tel que ceux décrits dans la demande de brevet FR 0955344), l'extrait des graines d'*Acacia macrostachya* (tel que celui décrit dans la demande de brevet FR 0958525) et des graines de *Vigna unguiculata* (tel que celui décrit dans la demande de brevet FR 0958529) ;
- les sucres de végétaux, en particulier les sucres d'avocat (tels que ceux décrits dans la demande WO2005/115421), utiles notamment pour leur propriété kératorégulatrice, cicatrisante et/ou restructurante de la barrière cutanée, anti-inflammatoire et/ou anti-irritante et/ou apaisante ;
- le butyle avocadate (5 alpha Avocuta®), inhibiteur de la 5-alpha réductase (voir WO 01/52837 et WO 02/06205) et typiquement, régulateur de la sécrétion séborrhée se trouvant augmentée dans l'acné ou les pellicules ;
- les extraits riches en polyphénol, et plus particulièrement les extraits de fruits d'avocat (tels que ceux décrits dans la demande FR 1 061 055) et les extraits de feuilles de Maca (tel que ceux décrits dans la demande FR 1 061 047) ;
- le lupéol (FR 2 822 821, FR 2 857 596) utile notamment pour favoriser la cicatrisation ;
- un extrait total de lupin (tel que ceux décrits dans la demande internationale WO2005/102259), particulièrement adapté pour le traitement des irritations ;
- un beurre de Cupuaçu, particulièrement apprécié pour ses propriétés hydratantes.

Parmi les actifs recommandés en association avec l'extrait selon l'invention, on peut citer les oxazolines, en particulier celles choisies dans le groupe constitué par la 2-undécyl-4-hydroxyméthyl-4-méthyl-1,3-oxazoline, la 2-undécyl-4,4-diméthyl-1,3-oxazoline, la (E)-4,4-diméthyl-2-heptadéc-8-ényl-1,3-oxazoline, la 4-hydroxyméthyl-4-méthyl-2-heptadécyl-1,3-oxazoline, la (E)-4-hydroxyméthyl-4-méthyl-2-heptadéc-8-ényl-1,3-oxazoline, la 2-undécyl-4-éthyl-4-hydroxyméthyl-1,3-oxazoline, de préférence la 2-undécyl-4,4-diméthyl-1,3-oxazoline (OX-100 ou Cyclocéramide® ; WO2004050052, WO2004050079, et WO2004112741). Elles sont particulièrement utiles pour leur activité anti-inflammatoire et/ou anti-irritante et/ou apaisante, antioxydante, dépigmentante, immunomodulatrice.

Parmi les actifs recommandés en association avec l'extrait selon l'invention, on peut citer les inhibiteurs de 5-alpha réductase, tels que le butyle avocadate (5 alpha Avocuta®).

Toutes ces associations comprennent au moins un autre composé actif, en plus de l'extrait de parties aériennes de *Gynandropsis gynandra,* et peuvent comprendre deux, trois, quatre ou plus de composés actifs tels que décrits précédemment.

La composition selon l'invention peut être formulée sous la forme de différentes préparations adaptées à une administration topique, à une administration orale, rectale, vaginale, nasale, auriculaire ou bronchique, ainsi qu'à une administration parentérale. La composition selon l'invention est avantageusement formulée sous la forme de différentes préparations adaptées à une administration topique, plus particulièrement pour application sur la peau, et/ou les phanères et/ou les muqueuses.

Selon une première variante, les différentes préparations sont adaptées à l'administration topique et incluent notamment les crèmes, les émulsions, les laits, les pommades, les lotions, les huiles, les solutions aqueuses ou hydro-alcooliques ou glycoliques, les poudres, les patchs, les sprays, les shampooings, les vernis ou tout autre produit pour application externe.

Les modes d'administration, les posologies et les formes galéniques optimales des composés et compositions selon l'invention peuvent être déterminés selon les critères généralement pris en compte dans l'établissement d'un traitement pharmaceutique, en particulier dermatologique, cosmétique ou vétérinaire adapté à un patient ou à un animal comme par exemple l'âge ou le poids corporel du patient ou de l'animal, la gravité de son état général, la tolérance au traitement, les effets secondaires constatés, le type de peau. En fonction du type d'administration souhaitée, la composition et/ou les composés actifs selon l'invention peuvent en outre comprendre au moins un excipient pharmaceutiquement acceptable, notamment dermatologiquement acceptable, ou un excipient cosmétiquement acceptable. Selon la première variante, on utilise un excipient adapté pour une administration par voie topique externe. La composition selon la présente invention peut en outre comprendre au moins un adjuvant pharmaceutique ou cosmétique connu de l'homme du métier, choisi parmi les épaississants, les conservateurs, les parfums, les colorants, des filtres chimiques ou minéraux, les agents hydratants, les eaux thermales, etc.

La composition comprenant un extrait de parties aériennes de *Gynandropsis gynandra* ayant les spécifications indiquées est particulièrement destinée à une utilisation cosmétique ou dermatologique. La composition sera avantageusement formulée sous la forme d'une préparation adaptée à une administration topique.

L'invention a également pour objet la composition selon l'invention ou un extrait de parties aériennes de *Gynandropsis gynandra* tel que défini ci-dessus, pour son utilisation en tant que composition cosmétique, pharmaceutique, dermatologique, avantageusement cosmétique ou dermatologique.

Avantageusement, la composition ou l'extrait selon la présente invention est utilisée dans la prévention et/ou le traitement des troubles ou pathologies de la peau et/ou des muqueuses et/ou des phanères.

En particulier, la composition ou l'extrait selon la présente invention est utilisée dans la prévention et/ou le traitement des troubles liés à l'acné.

La physiopathologie de l'acné est associée à différents facteurs déclenchant parmi lesquels : l'hyper-séborrhée, l'hyper-kératinisation folliculaire (comédogénèse), et la colonisation bactérienne par *Propionibacterium acnes* (*P. acnes*)*.* Ces facteurs sont intriqués et interagissent les uns avec les autres (Bellew *et al.* 2011, 10(6) : 582-585).

La production de sébum par les glandes sébacées joue un rôle crucial dans le développement des lésions d'acné. En effet, l'hyper-séborrhée associée à l'altération qualitative du sébum engendre des signaux pouvant induire l'hyper-kératinisation et créer, ainsi, un environnement propice à la multiplication de *P. acnes.*

L'acné est aussi associée à un développement excessif de *P. acnes.* Cette bactérie commensale se conduit comme un pathogène opportuniste favorisé par l'environnement de l'acné (hyper-séborrhée / inflammation / obstruction du follicule). *P. acnes* produit de nombreux facteurs qui contribuent à aggraver la comédogénèse et l'inflammation : signalisation via TLR (récepteurs de type Toll) pour induire et maintenir l'inflammation, relargage d'enzymes conduisant à la rupture de la paroi du follicule, production de facteurs chimiotactiques pour l'attraction du neutrophile.

La composition ou l'extrait selon la présente invention est donc également utilisée dans la prévention et/ou le traitement des troubles liés à l'hyperséborrhée, tels que la dermite séborrhéique (croutes de lait), l'acné et les peaux à tendance acnéiques, et les états pelliculaires.

La composition ou l'extrait selon l'invention est particulièrement utile dans la prévention et/ou le traitement des troubles vasculaires, pour la protection des vaisseaux sanguins et/ou pour agir sur la circulation sanguine, en particulier la microcirculation sanguine.

La composition ou l'extrait selon l'invention est donc avantageusement utilisée dans la prévention et/ou le traitement de la dilatation (chronique) des capillaires sous-cutanés qui peut se produire dans les conditions telles que la couperose, les érythèmes cutanés, la rosacée, le prurit, la peau et/ou les muqueuses réactives, avec des rougeurs, en particulier dues à une dilatation des capillaires sous-cutanés.

La composition ou l'extrait selon l'invention est également avantageusement utilisé(e) en tant que produit anti-âge chronologique ou photo-induit dans la prévention du vieillissement, et du vieillissement photo-induit et, en tant que produit cicatrisant, dans la prévention et/ou le traitement des troubles liés à la cicatrisation et à l'organisation cutanée.

On entend par troubles liés à la cicatrisation et à l'organisation cutanée, les troubles résultant des processus de cicatrisation et d'organisation cutanée de la peau telles qu'une peau relâchée, des vergetures, des dartres, des gerçures, des crevasses en particulier au niveau des seins.

En particulier, la composition ou l'extrait selon l'invention est utilisé(e) en tant que produit hydratant, intervenant dans la prévention et/ou le traitement des troubles de la barrière ou de l'homéostasie de la peau, des phanères (cheveux et ongles) et/ou des muqueuses (gencives, parodontes, muqueuses génitales) immature(s), normale(s) ou mature(s)/âgée(s).

On entend par troubles de la barrière de la peau, des phanères et/ou des muqueuses les troubles intervenant au niveau de la couche extérieure de l'épiderme.

On entend par troubles de l'homéostasie de la peau, des phanères et/ou des muqueuses les troubles résultant des processus de renouvellement et d'équilibre des cellules telles que le psoriasis, la dermite du siège, la dermatite atopique, la peau sèche (xérose), la peau déshydratée et la peau photosensibilisée.

La composition ou l'extrait selon l'invention est également avantageusement utilisé(e) dans le traitement et/ou la prévention des inflammations dues aux rayons de toutes sortes, en particulier des coups de soleil.

La composition ou l'extrait selon la présente invention peut également être avantageusement utilisé(e) comme produit amincissant dans la régulation du tissu adipeux, de la cellulite et plus particulièrement via l'inhibition de la lipogénèse.

La composition ou l'extrait selon l'invention est utilisé(e) comme produit anti-allergique dans la prévention et/ou au traitement des réactions ou pathologies allergiques telle que la dermatite allergique, la dermatite de contact, l'eczéma et le prurit.

Avantageusement, la composition ou l'extrait selon l'invention peut être utilisé(e) en tant que produit pro-pigmentant dans la prévention et/ou le traitement des réactions, troubles ou pathologies de la peau présentant des troubles de la dépigmentation, telle que la peau dépigmentée (vitiligo).

Avantageusement, la composition ou l'extrait selon l'invention peut être utilisé(e) pour la prévention et/ou le traitement des réactions, troubles ou pathologies de la peau ou des réactions, troubles ou pathologies des phanères tels que les cheveux (alopécie, pellicules, hirsutismes, folliculites) ou des réactions, troubles ou pathologies des muqueuses telles que les gencives et parodontes pouvant présenter des gingivites (gencives sensibles des nouveaux nés, problèmes d'hygiène, dus au tabagisme ou autres), des parodontopathies, ou des muqueuses génitales pouvant présenter des irritations des sphères génitales males ou femelles externes ou internes, liés à un déficit de l'immunité innée (peptides antimicrobiens) ou acquise (cellulaire, humorale, cytokines)

L'invention concerne également un procédé de soin cosmétique de la peau et/ou des phanères et/ou des muqueuses, en vue d'améliorer leur état et/ou leur aspect, consistant à appliquer sur la peau et/ou phanères et/ou muqueuses des patients qui en ont besoin une composition ou un extrait selon la présente invention.

Dans un mode de réalisation du procédé cosmétique selon l'invention, la peau et/ou les phanères et/ou les muqueuses visées sont avantageusement celles qui sont sensibles, irritées, agressées par l'environnement (UV, pollution), en particulier les peaux sensibles. Les peaux sensibles présentent souvent des rougeurs (notamment du visage), typiquement avec des tiraillements ou des picotements quotidiens.

Le procédé cosmétique selon l'invention est en particulier en vue d'améliorer les patients souffrant d'acné et présentant des peaux à tendance acnéique et également en vue d'améliorer les états pelliculaires.

Le procédé cosmétique selon l'invention est également caractérisé en ce que la composition ou l'extrait est un produit anti-âge chronologique ou photo-induit, hydratant, amincissant et/ou anticellulite.

### Exemple comparatif 1

Les tiges feuillées de *Gynandropsis gynandra* séchées et broyées sont mises en suspension sous agitation à 5% dans un mélange éthanol/eau 80/20 p/p pendant 1h à température ambiante. La matière sèche résiduelle est séparée de la phase liquide soit par filtration, décantation ou centrifugation et la phase liquide ainsi obtenue peut être filtrée à l'aide de filtres de porosité adaptée afin d'obtenir une solution limpide. L'extrait obtenu présente les caractéristiques suivantes :
▪ Sucres totaux (anthrone): 41% /sec
▪ Polyphénols totaux (Folin - Ciocalteu) - éq. Acide gallique : 7,7% / sec
▪ Protéines (dosage de Bradford) : 3,1%/sec.

Cet extrait présente une activité anti-radicalaire, anti-DPPH « in tubo », pour laquelle la concentration inhibitrice 50 (IC50) a pu être déterminée et est de 0,2 mg d'extrait sec, ce qui représente 19 µg de polyphénols dans le milieu réactionnel.

### Exemple 2

Les tiges feuillées de *Gynandropsis gynandra* séchées et broyées sont mises en suspension sous agitation à 5% dans un mélange glycérol/eau 80/20 p/p pendant 1 h à température ambiante. La matière sèche résiduelle est séparée de la phase liquide soit par filtration, décantation ou centrifugation et la phase liquide ainsi obtenue peut être filtrée à l'aide de filtres de porosité adaptée afin d'obtenir une solution limpide. L'extrait obtenu présente les caractéristiques suivantes :
▪ Sucres totaux (anthrone) : 65% /sec
▪ Polyphénols totaux (Folin - Ciocalteu) - éq. Acide gallique : 6,4% /sec
▪ Teneurs en flavonoïdes (AlCl3) - éq. rutine : 4% / sec
▪ Protéines (dosage de Bradford) : 4,0% /sec.

Cet extrait présente une activité anti-radicalaire, anti-DPPH « in tubo », pour laquelle la concentration inhibitrice 50 (IC50) a pu être déterminée et est de 0,09 mg d'extrait sec, ce qui représente 7,1 µg de polyphénols dans le milieu réactionnel.

### Exemple 3

Les tiges feuillées de *Gynandropsis gynandra* séchées et broyées sont mises en suspension sous agitation à 5% dans un mélange propanediol/eau 60/40 p/p pendant 1 h à température ambiante. La matière sèche résiduelle est séparée de la phase liquide soit par filtration, décantation ou centrifugation et la phase liquide ainsi obtenue peut être filtrée à l'aide de filtres de porosité adaptée afin d'obtenir une solution limpide. L'extrait obtenu présente les caractéristiques suivantes :
▪ Sucres totaux (anthrone) : 32% /sec
▪ Polyphénols totaux (Folin - Ciocalteu) - éq. acide gallique : 7,6% / sec
▪ Teneurs en flavonoïdes (AlCl3) - éq. rutine : 4,8% / sec
▪ Protéines : 1%.

Cet extrait présente une activité anti-radicalaire, anti-DPPH « in tubo », pour laquelle la concentration inhibitrice 50 (IC50) a pu être déterminée et est de 0,16 mg d'extrait sec, ce qui représente 14 µg de polyphénols dans le milieu réactionnel.

### Exemple 4 : Compositions pour application par voie topique

Les inventeurs présentent ci-dessous plusieurs compositions pour application par voie topique. Les extraits de parties aériennes de *Gynandropsis gynandra* peuvent être incorporés à divers produits cosmétiques tels que des eaux nettoyantes, des émulsions huile dans eau, des émulsions eau dans huile, des huiles, des laits, des lotions, des shampooings, des produits moussants et sprays, dont les compositions sont présentées ci-dessous. Les pourcentages représentent le poids du produit par rapport au poids total de la composition.

### EAU NETTOYANTE PEAU SENSIBLE

| **Nom commercial ou INCI** | **%** |
|---|---|
| CAPRYLOYL GLYCINE | De 0 à 1 % |
| LESSIVE SOUDE | De 0 à 1 % |
| SEQUESTRANT | De 0 à 1 % |
| BUTYLENE GLYCOL | De 1 à 5 % |
| BETA CAROTENE | De 0 à 2 % |

| **Extrait de Gynandropsis gynandra** | De 0,01 à 10 % |
|---|---|
| CONSERVATEURS | De 0 à 1 % |
| PEG-32 | De 1 à 5 % |
| PEG-7 PALMCOCOATE | De 1 à 5 % |
| GLUCONATE ZINC | De 0 à 1 % |
| ACIDE CITRIQUE | De 0 à 1 % |
| EAU PURIFIÉE | QSP 100 % |
| PARFUM | De 0 à 1 % |
| POLOXAMER 184 | De 1 à 5 % |

### EMULSION ANTI-AGE

| **Nom commercial ou INCI** | **%** |
|---|---|
| ISOPARAFFINE LIQUIDE | De 5 à 20 % |
| STEARATE D'ISOCETYLE | De 5 à 20 % |
| HYDROXYSTEARATE AL - MG | De 5 à 20 % |
| ABIL WE 09 | De 1 à 5 % |
| GLYCEROL | De 1 à 5 % |
| HUILE VASELINE | De 1 à 5 % |
| ZINC OXYDE MICRONISE | De 1 à 5 % |
| BUTYLENE GLYCOL | De 1 à 5 % |
| RETINOL | De 0 à 1% |
| VITAMINE C | De 0 à 5% |
| **Extrait de Gynandropsis gynandra** | De 0,01 à 10 % |
| ISONONYL ISONONANOAT | De 1 à 5 % |
| CIRE D'ABEILLE | De 1 à 5 % |
| TARTRATE DE SODIUM | De 1 à 5 % |
| CHLORURE DE SODIUM | De 0 à 5 % |
| GLYCINE | De 1 à 5 % |
| CONSERVATEURS | De 0 à 1 % |
| CHOLESTEROL | De 0 à 1 % |
| PHYTOSPHINGOSINE | De 0 à 1 % |
| ACIDE TARTRIQUE | De 0 à 1 % |
| EAU PURIFIÉE | QSP 100 % |

### EMULSION ANTI-ROUGEURS

| **Matière première / Nom commercial ou INCI** | **%** |
|---|---|
| PEG 40 STEARATE | De 1 à 5 % |
| PEG 5 GLYCERYL STEAR | De 1 à 5 % |
| CERESINE WAX | De 1 à 5 % |
| MONOSTEARATE DE GLYCEROL | De 1 à 5 % |
| STEARATE DE SORBITAN | De 0 à 2 % |
| ALCOOL CETYLIQUE | De 0 à 2 % |
| ALCOOL DI-MALATE | De 5 à 20 % |
| ESCULOSIDE | De 0 à 2 % |
| SOPHORA JAPONICA | De 0 à 5 % |
| VITAMINE E | De 0 à 1 % |
| **Extrait de Gynandropsis gynandra** | De 0,01 à 10 % |
| BUTYLENE GLYCOL | De 1 à 5 % |
| PIROCTOLAMINE | De 0 à 1 % |
| CONSERVATEURS | De 0 à 1 % |
| GLYCEROL | De 1 à 10 % |
| GOMME XANTHANE | De 0 à 1 % |
| ZINC PCA | De 0 à 2 % |
| AMIDON DE RIZ | De 1 à 5 % |
| NYLON 6 | De 0 à 2 % |
| POLYACRYLAMIDE GEL | De 1 à 5 % |
| VITAMINE B6 | De 0 à 1 % |
| PARFUM | De 0 à 1 % |
| EAU PURIFIÉE | QSP 100 % |

### SPRAY SOLAIRE SPF 50+

| **Matière première / Nom commercial ou INCI** | **%** |
|---|---|
| CAPRYLO CAPRATE DE GLYCEROL | De 5 à 20% |
| CYCLOPENTASILOXANE | De 10 à 20% |
| DICAPRYLYL CARBONATE | De 5 à 20% |
| TINOSORB S | De 1 à 10% |
| OXYDE DE TITANE 100 | De 10 à 20% |
| HECTORITE | De 0 à 5% |
| ALPHA TOCOPHEROL | De 0 à 2% |
| LAURYLGLUCOSIDE-GLYSTEARATE | De 0 à 100% |
| EAU PURIFIÉE B4 | QSP 100% |
| ACIDE CITRIQUE | De 0 à 2% |
| PENTYLENE GLYCOL | De 0 à 5% |
| GLYCEROL | De 0 à 5% |
| GOMME XANTHANE | De 0 à 2% |
| **Extrait de Gynandropsis gynandra** | **De 0,01 à 10 %** |
| ALOE VERA | De 0 à 1% |
| GLUCONATE ZINC | De 0 à 1% |
| CONSERVATEURS | De 0 à 2% |
| TINOSORB M | De 1 à 10% |

### EMULSION ANTI-ACNEIQUE

| **Matière première / Nom commercial ou INCI** | **%** |
|---|---|
| PEG 40 STEARATE | De 1 à 5 % |
| PEG 5 GLYCERYL STEAR | De 1 à 5 % |
| CERESINE WAX | De 1 à 5 % |
| MONOSTEARATE DE GLYCEROL | De 1 à 5 % |
| STEARATE DE SORBITAN | De 0 à 2 % |
| ALCOOL CETYLIQUE | De 0 à 2 % |
| ALCOOL DI-MALATE | De 5 à 20 % |
| VITAMINE E | De 0 à 1 % |
| VITAMINE B3 | De 0 à 5 % |
| ACIDE LINOLEIQUE | De 0 à 1 % |
| **Extrait *Gynandropsis Gynandra*** | De 0,01 à 10 % |
| BUTYLENE GLYCOL | De 1 à 5 % |
| PIROCTOLAMINE | De 0 à 1 % |
| CONSERVATEURS | De 0 à 1 % |
| GLYCEROL | De 1 à 10 % |
| GOMME XANTHANE | De 0 à 1 % |
| ZINC PCA | De 0 à 2 % |
| AMIDON DE RIZ | De 1 à 5 % |
| NYLON 6 | De 0 à 2 % |
| POLYACRYLAMIDE GEL | De 1 à 5 % |
| VITAMINE B6 | De 0 à 1 % |
| PARFUM | De 0 à 1 % |
| EAU PURIFIÉE | QSP 100 % |

### SHAMPOOING ANTIPELLICULAIRE

| **Matière première / Nom commercial ou INCI** | **%** |
|---|---|
| EAU PURIFIÉE | QSP 100 % |
| LAUROAMPHOACETATE | De 5 à 20 % |
| COCOGLUCOSIDE | De 5 à 20 % |
| DISTEARATE DE PEG 6000 | De 1 à 5 % |
| CONSERVATEURS | De 0 à 2 % |
| VITAMINE F | De 0 à 5 % |
| PIROCTONE OLAMINE | De 0 à 2 % |
| **Extrait de Gynandropsis gynandra** | De 001 à 10 % |
| ZINC PYRITHIONE | De 0 à 1 % |
| AJUSTEUR pH | De 0 à 1 % |
| SEQUESTRANT | De 0 à 1 % |
| PARFUM | De 0 à 1 % |

### Exemple 5 : Activité biologique de l'extrait de Gynandropsis gynandra

### A. Activité biologique dans l'acné

### A. 1.Action sur l'hyper-séborrhée : Inhibition de la synthèse de lipides par des sébocytes

### - Matériel et méthodes :

Des sébocytes humains (lignée SZ95), stimulés ou non par l'acide arachidonique à 50µM, ont été traités par l'extrait de *Gynandropsis gynandra* (GG) à 0,005% ; 0,01% et 0,02% (p/v de matière active) ou par un inhibiteur de référence pendant 24 ou 48 heures.

A l'issue du traitement, les lipides neutres ont été quantifiés par mesure de fluorescence après marquage au rouge de Nile. Cette mesure, exprimée en unité de fluorescence (RFU) reflète la synthèse « *de novo* » de lipides intracellulaires par les sébocytes.

Les résultats ont été analysés statistiquement par une analyse de variance (ANOVA) à un facteur suivie d'un test de Dunnett.

### - Résultats et conclusion :

L'extrait de GG a fortement et significativement inhibé la production de lipides par des sébocytes en condition basale (synthèse constitutive) et stimulés par l'acide arachidonique (tableau 1).

Ces résultats montrent que l'extrait de GG présente un intérêt pour réguler la production de sébum qui est accrue dans l'acné.

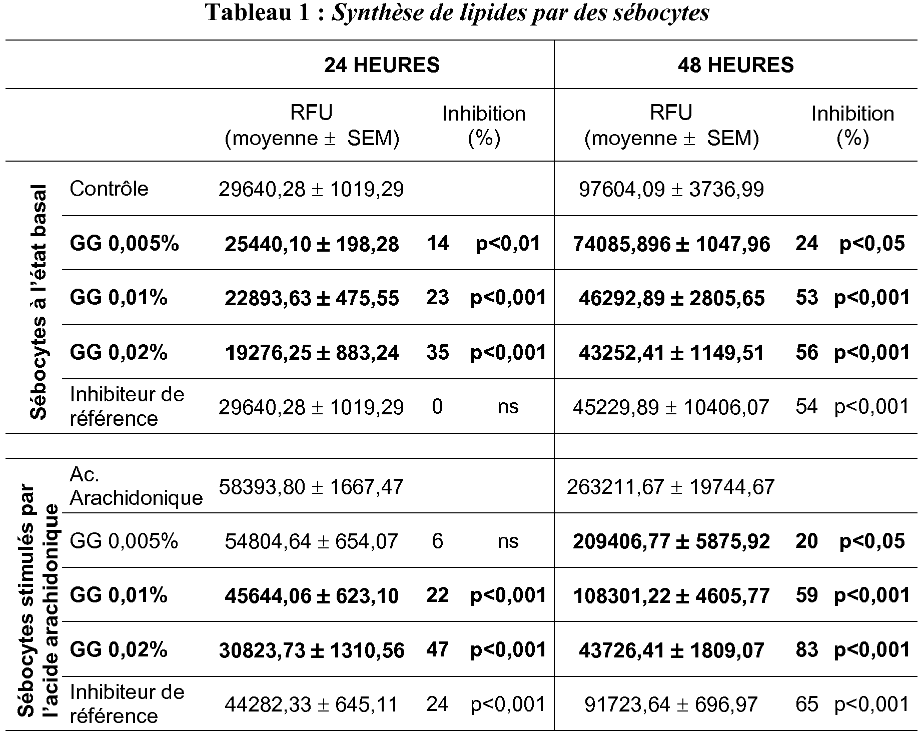

### A.2. Action sur les facteurs aggravants de l'acné

### 1. Modèle de kératinocytes

### - Matériel et méthodes :

Des kératinocytes humains (lignée NCTC-2544) ont été pré-incubés ou non (contrôle) par l'extrait de *Gynandroposis gynandra* (GG) à 0,01% et 0,02% (p/v de matière active) ou les molécules de référence (dexaméthasone à 10⁻⁷M ; indométhacine à 10⁻⁶M) pendant 24 heures. Les cellules ont ensuite été traitées par le PMA à 0,1 µg/ml (Phorbol Myristate Acetate) pendant 24 heures, toujours en présence de GG ou des références.

A l'issue du traitement, les quantités d'IL8 (interleukine 8) et de PGE2 (prostaglandine E2) sécrétées ont été mesurées par ELISA dans les surnageants de culture. Les résultats ont été analysés statistiquement par un test t de Student.

### - Résultats et conclusion :

L'extrait de GG a fortement et significativement inhibé la production des médiateurs aggravants de l'acné IL8 et PGE2 stimulée par le PMA dans des kératinocytes (tableau 2).

L'extrait de GG module les facteurs aggravants précoces de l'acné.

**Tableau 2 : Production d'IL8 et de PGE2 par des kératinocytes**

| | **IL8** (ng/ml) | Inhibition | |
|---|---|---|---|
| Cellules contrôles | 0,1 ± 0,0 | | |
| PMA 0,1 µg/ml | 50,1 ± 1,8 | | |
| Dexaméthasone 10-7M | 7,4 ± 0,8 | 85% | p<0,001 |
| **GG 0,01%** | **28,3 ± 0,5** | **44%** | p<0,001 |
| **GG 0,02%** | **17,6 ± 0,5** | **65%** | p<0,001 |
| | | | |

| | **PGE2** (ng/ml) | Inhibition | |
|---|---|---|---|
| Cellules contrôles | 0,039 ± 0,0 | | |
| PMA 0,1 µg/ml | 138,4 ± 10,6 | | |
| Indométhacine 10-6M | 0,039 ± 0,0 | 100% | p<0,001 |
| **GG 0,01%** | **24,1 ± 3,0** | **83%** | p<0,001 |
| **GG 0,02%** | **9,8 ± 0,0** | **93%** | p<0,001 |

### 2. Modulation de l'effet de P. acnes sur des kératinocytes

### - Matériel et méthodes :

Des kératinocytes humains (lignée HaCaT) ont été pré-incubés pendant 48h en présence de l'extrait de *Gynandropsis gynandra* (GG) à 0,0005% ; 0,002% ; 0,008% et 0,031% (p/v de matière active) ou de l'inhibiteur de référence : nicotinamide.

Les kératinocytes ont alors été stimulés par incubation pendant 18 heures avec une suspension bactérienne de *P. acnes* (souche ATCC6919).

A la fin de l'incubation, la quantité d'IL8 produite par les kératinocytes a été mesurée dans les surnageants de culture par une technique ELISA.

Les résultats ont été analysés statistiquement par un test t de Student : ns p>0,05 (non significatif) ; *p<0,05 ; **p<0,01 ; ***p<0,001.

### - Résultats et conclusion :

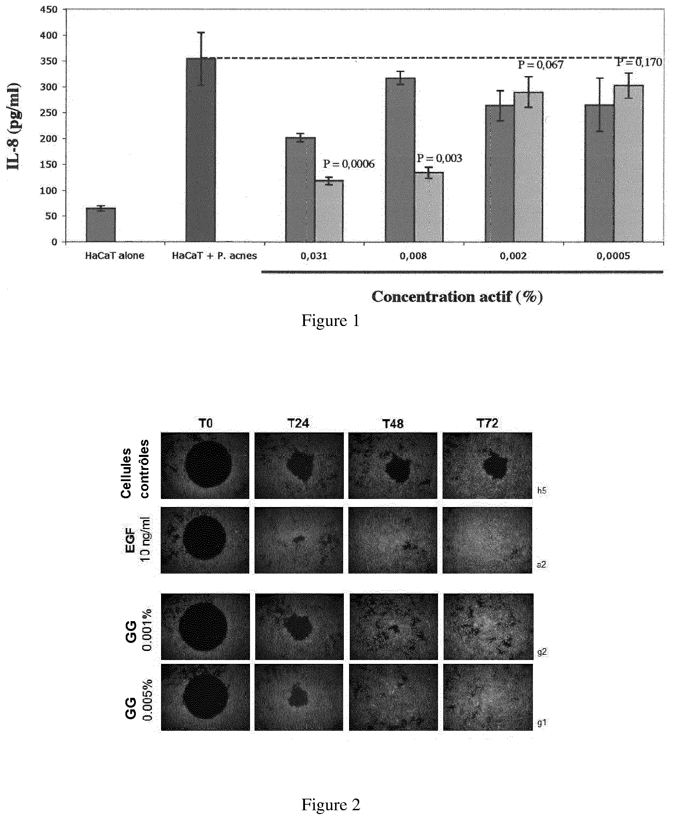

La figure 1 représente la production d'IL8, en pg/ml, par des kératinocytes stimulés par *P.acnes*, en fonction de la concentration d'actif en %.

L'extrait de GG a significativement inhibé la production d'IL8 induite par *P. acnes* sur des kératinocytes (figure 1).

L'extrait de GG module l'impact de *P. acnes* dans la physiopathologie de l'acné.

### 3. Stimulation de l'expression de peptides anti-microbiens

### - Matériel et méthodes :

Des kératinocytes humains normaux, cultivés dans du milieu enrichi en Ca⁺⁺, ont été traités pendant 24 heures par l'extrait de *Gynandropsis gynandra* (GG) à 0,005% (p/v de matière active).

A la fin du traitement, l'expression génique des peptides anti-microbiens (beta-défensines 2 et 3 -hBD2, hBD3- et cathélicidine LL37) a été analysée par RT-PCR quantitative en temps réel.

Les résultats ont été analysés statistiquement par une ANOVA à un facteur suivie d'un test de Dunnett : ns p>0,05 (non significatif) ; ** p<0,01 ; ***p<0,001.

### - Résultats et conclusion :

L'extrait de GG a stimulé l'expression des peptides anti-microbiens par les kératinocytes (tableau 3).

Ainsi, l'extrait de GG permet de limiter la colonisation bactérienne de la peau et donc de limiter la pathogénicité liée à *P. acnes.*

**Tableau 3 : Expression génique des peptides anti-microbiens hBD2, hBD3, LL37 dans des kératinocytes (Quantité Relative)**

| | **HBD2** | **HBD3** | **LL37** |
|---|---|---|---|
| Cellules contrôles | 1,00 | 1,00 | 1,00 |
| GG 0,005% | **2,70 (+170% **)** | **1,85 (+85% ***)** | **1,51 (+51% ns)** |

### 4. Modulation de la migration de neutrophiles

Le neutrophile, joue un rôle important dans la physiopathologie de l'acné. Il est présent en forte quantité dans la peau acnéique notamment en raison des nombreuses substances chimiotactiques produites par *P. acnes.*

### - Matériel et méthodes :

Des neutrophiles humains ont été pré-traités pendant 30 minutes par l'extrait de *Gynandropsis gynandra* à 0,001% et 0,002% (p/v de matière active) ou par un inhibiteur de référence.

Les cellules ont alors été déposées dans le système de migration « Transwell ® » : les neutrophiles pré-traités ont été déposés dans des inserts positionnés sur une plaque réceptrice contenant le chemoattractant : fMLP (N-formyl-Met-Leu-Phe) à 1 µM. Après 2 heures d'incubation, le nombre de neutrophiles ayant migré a été évalué par mesure de l'activité enzymatique de la LDH (Lactate Déshydrogénase).

Les résultats ont été analysés statistiquement par un test t de Student.

### - Résultats et conclusion :

La migration des neutrophiles a été significativement inhibée par l'extrait de GG (tableau 4).

L'extrait de GG empêche donc le recrutement de neutrophiles dans la peau décrit dans l'acné.

**Tableau 4 : Migration de neutrophiles**

| | **Migration** (%) | Inhibition par rapport aux cellules stimulées par fMLP |
|---|---|---|
| Cellules contrôles | 1,89 ± 0,2 | |
| fMLP | 25,1 ± 3,02 | |
| Inhibiteur | 6,4 ± 0,26 | -74% (p<0,01) |
| **GG 0,001%** | **8,65 ± 0,24** | **-66% (p<0,01)** |
| **GG 0,002%** | **14,05 ± 1,26** | **-44% (p<0,05)** |

### 5. Inhibition de la production de leukotriène B4 par des neutrophiles

Le leukotriène B4 est produit et libéré massivement par le neutrophile, il joue un rôle dans les lésions d'acné et induit la sécrétion de sébum.

### - Matériel et méthodes :

Des neutrophiles humains ont été pré-incubés pendant 15 minutes en présence de l'extrait de *Gynandropsis gynandra* (GG) à 0,002% et 0,008% (p/v de matière active). Les cellules ont ensuite été stimulées par ajout de zymosan opsonisé à 1 mg/ml.

Après 10 minutes d'incubation, le leukotriène B4 (LTB4) libéré par les cellules a été dosé dans les surnageants de cellules par une technique ELISA.

Les résultats ont été analysés statistiquement par un test t de Student.

### - Résultats et conclusion :

L'extrait de GG a significativement inhibé la production de LTB4 induite par le zymosan opsonisé sur des neutrophiles (tableau 5).

Ainsi, l'extrait de GG module l'impact du neutrophile et de LTB4 dans l'acné.

**Tableau 5 : Production de leukotriène B4 par des neutrophiles**

| | **LTB4** (pg/ml) | Inhibition | |
|---|---|---|---|
| Cellules contrôles | 81,45 ± 7,16 | | |
| Cellules stimulées | 439,32 ± 3,01 | | |
| **GG 0,002%** | **199,17 ± 4,83** | **-54%** | **p<0,001** |
| **GG 0,008%** | **220,91 ± 1,75** | **-49%** | **p<0,001** |

### 6. Inhibition de la libération d'histamine par des mastocytes

L'histamine peut altérer la production du sébum. En effet, les sébocytes (cellules constitutives de la glande sébacée, responsables de la production du sébum) présentent à leur surface des récepteurs à l'histamine.

### - Matériel et méthodes :

Des mastocytes ont été pré-incubés pendant 30 minutes en présence de l'extrait de *Gynandropsis gynandra* (GG) à 0,08% (p/v de matière active) ou de calcium à 10 mM (inhibiteur de référence de la libération d'histamine).

Les mastocytes ont ensuite été stimulés par la substance P à 10 µM pendant 15 minutes. A la fin de l'incubation, l'histamine libérée a été quantifiée par ELISA.

Les résultats ont été analysés statistiquement par un test t de Student.

### - Résultats et conclusion :

L'extrait de GG a fortement et significativement inhibé la libération d'histamine par des mastocytes stimulés par la substance P (tableau 6).

L'extrait de GG module l'effet de l'histamine dans l'acné.

**Tableau 6 : Libération d'histamine par des mastocytes**

| | **Histamine** (ng/ml) | Inhibition | |
|---|---|---|---|
| Contrôle | 20,1 ± 1,9 | | |
| Substance P | 142,5 ± 9,6 | | |
| Calcium 10 mM | 21,8 ± 1,0 | -85% | p<0,01 |
| **GG 0,08%** | **12,3 ± 1,3** | **-91%** | **p<0,01** |

### A.3. Stress oxydant

Dans l'acné, il y a un stress oxydant caractérisé par une forte production de radicaux libres oxygénés (RLO). La sévérité de l'acné est corrélée à cette quantité de RLO.

Par ailleurs, le squalène (constituant lipidique du sébum) est oxydé dans l'acné.

### 1. Effet anti-oxydant

### - Matériel et méthodes :

Des kératinocytes humains normaux ont été traités pendant 24 heures par l'extrait de *Gynandropsis gynandra* (GG) à 0,032% (p/v de matière active) ou la vitamine C à 10 µg/ml (référence anti-oxydante) avant incorporation de la sonde H2DCF-DA (incubation de 45 minutes).

Les kératinocytes ont ensuite été stimulés par du peroxyde d'hydrogène (H₂O₂) à 100 µM pendant 20 minutes.

La production de ROS (Reactive Oxygen Species) a été évaluée par mesure de fluorescence.

Les résultats ont été analysés statistiquement par un test t de Student.

### - Résultats et conclusion :

L'extrait de GG a inhibé la production de ROS par des kératinocytes en réponse à un stress oxydant induit par H2O2 (tableau 7), il présente donc un effet anti-oxydant.

**Tableau 7 : Production de ROS dans des kératinocytes traités par H₂O₂**

| | **ROS** (Unités de fluorescence) | Inhibition | |
|---|---|---|---|
| Cellules stimulées (H₂O₂) | 1474,5 ± 93,32 | | |
| Référence (Vit. C) | 992,83 ± 93,96 | 33% | p<0,05 |
| **GG 0,032%** | **981,33 ± 132,52** | **33%** | **p<0,05** |

### 2. Protection contre la peroxydation lipidique

### - Matériel et méthodes :

Des cellules de lignée Jurkat ont été pré-incubées pendant 45 minutes en présence de l'extrait de *Gynandropsis gynandra* (GG) à 0,02% (p/v de matière active) ou du BHT à 100 µM (référence) et en présence de la sonde fluorescente C11-fluor, spécifique de la peroxydation des lipides.

Les cellules ont ensuite été irradiées par des UVA+B puis incubées pendant 30 minutes en présence de GG ou du BHT.

A la fin de l'incubation, la quantité de peroxydes lipidiques a été évaluée par analyse en cytométrie en flux de l'intensité de fluorescence (inversement proportionnelle à l'oxydation).

Les résultats ont été analysés statistiquement par un test t de Student.

### - Résultats et conclusion :

L'extrait de GG a significativement protégé les cellules contre la peroxydation lipidique induite par l'irradiation aux UV (tableau 8).

**Tableau 8 : Peroxydation des lipides induite par UV**

| | **Peroxydes lipidiques** (% du témoin irradié) | Protection (%) | |
|---|---|---|---|
| Cellules irradiées (UV) | 100 | | |
| BHT 100 µM | 64 | 49% | p < 0,01 |
| **GG 0,02%** | **83** | **23%** | **p < 0,05** |

### A.4. Action cicatrisante : stimulation de la migration kératinocytaire

Les lésions d'acné peuvent conduire à la formation de cicatrices disgracieuses.

Aussi, la prise en charge de l'acné peut être accompagnée d'une action pro-cicatrisante.

### - Matériel et méthodes :

Une plaie artificielle a été réalisée sur un tapis de kératinocytes humains normaux en monocouche.

Les cellules ont été marquées à la calcéine puis incubées pendant 72 heures en présence de l'extrait de *Gynandropsis gynandra* (GG) à 0,001% et 0,005% (p/v de matière active) ou par la référence (EGF à 10 ng/ml).

A 0, 24, 48 et 72 heures, la migration des kératinocytes a été suivie par microphotographie et quantifiée par mesure de surface de la plaie.

La figure 2 représente des photographies de cellules contrôles ou de cellules incubées en présence de l'extrait de gynandropsis gynandra (GG) à 0,001% et 0,005% (p/v de matière active) ou par la référence (EGF à 10 ng/ml) à 0 (T0), 24 (T24), 48 (T48) et 72 heures (T72) ; T0 étant l'instant où la plaie a été réalisée.

Les résultats ont été analysés statistiquement par un test t de Student.

### - Résultats et conclusion :

L'extrait de GG a significativement stimulé la migration des kératinocytes (tableau 9, figure 2) avec un recouvrement total de la plaie dès 48 heures.

L'extrait de GG favorise donc le processus de réépithélialisation au cours de la cicatrisation cutanée.

**Tableau 9 : Evaluation de la migration des kératinocytes**

| | **Zone de migration** (% de recouvrement de la plaie) | | |
|---|---|---|---|
| | **24h** | **48h** | **72h** |
| Cellules contrôles | 62 | 68 | 71 |
| Référence (EGF) | 95 (p<0,001) | 99 (p<0,001) | 100 (p<0,001) |
| **GG 0,001%** | **68 (ns)** | **96 (p<0,001)** | **99 (p<0,001)** |
| **GG 0,005%** | **72 (ns)** | **99 (p<0,001)** | **99 (p<0,001)** |

### B. Autres activités biologiques

### B.1. Inhibition de la lipogénèse dans des adipocytes

### - Matériel et méthodes :

Des adipocytes humains normaux ont été incubés pendant 1 heure en présence de l'extrait de *Gynandropsis gynandra* (GG) à 0,02% (p/v de matière active) ou de la référence (cérulénine à 20 µM). Après incubation, le marqueur radioactif [¹⁴C]-acétate a été ajouté et les échantillons ont été incubés la nuit.

A la fin de l'incubation, les lipides ont été extraits et la radioactivité incorporée (correspondant à la lipogénèse) a été mesurée par scintillation liquide.

Les résultats ont été analysés statistiquement par un test t de Student.

### - Résultats et conclusion :

L'extrait de GG a significativement inhibé la néosynthèse des lipides par des adipocytes (tableau 10).

Ainsi, cet extrait présente donc un effet amincissant.

**Tableau 10 : Evaluation de la lipogénèse dans des adipocytes**

| | **Incorporation d'acétate** (cpm) | Inhibition | |
|---|---|---|---|
| Cellules contrôles | 32895 ± 1358 | | |
| Référence (Cérulénine) | 14934 ± 671 | 55% | p<0,001 |
| **GG 0,02%** | **26573 ± 227** | **19%** | **p<0,05** |

### B.2. Augmentation de la production de mélanine par des mélanocytes

### - Matériel et méthodes :

Des mélanocytes épidermiques humains normaux ont été cultivés pendant 240 heures en présence de l'extrait de *Gynandropsis gynandra* (GG) à 0,002% et 0,01% (p/v de matière active) et de NDP-MSH à 10⁻⁷M (stimulation de la synthèse de mélanine).

Après incubation, la mélanine a été extraite des cellules et quantifiée par spectrophotométrie.

Les résultats ont été analysés statistiquement par un test t de Student.

### - Résultats et conclusion :

L'extrait de GG a significativement stimulé et potentialisé l'effet du NDP-MSH sur la production de mélanine (tableau 11).

L'extrait présente donc un effet pro-pigmentant.

**Tableau 11 : Production de mélanine par des mélanocytes**

| | **Mélanine** (µg/ml) | Stimulation | |
|---|---|---|---|
| Cellules contrôles | 13,0 ± 1,1 | | |
| Témoin stimulé (NDP-MSH) | 27,1 ± 0,9 | +108% | p<0,001 |
| **GG 0,002%** | **35,1 ± 0,3** | **+170%** | **p<0,01** |
| **GG 0,01%** | **41,2 ± 0,5** | **+217%** | **p<0,001** |

### B.3. Screening d'activité sur cellules endothéliales

### - Matériel et méthodes :

Des cellules endothéliales microvasculaires humaines ont été traitées par l'extrait de *Gynandropsis gynandra* (GG) à 0,01% et 0,02% (p/v de matière active) pendant 24 heures.

Après incubation, l'expression génique de différents marqueurs a été analysée par RT-PCR quantitative en temps réel à l'aide d'un PCR array.

### - Résultats et conclusion :

Les résultats du screening sur cellules endothéliales ont permis de montrer que l'extrait de *Gynandropsis gynandra* (tableau 12) :
- inhibe l'expression génique des molécules pro-angiogéniques : PGF (Placental Growth Factor), PDGF (Platelet Derived Growth Factor B subunit) et VEGFR3 (Vascular endothelial growth factor receptor 3).
- stimule l'expression de molécules anti-angiogéniques : thrombospondine-1 et endostatine.
- stimule l'expression de molécules impliquées dans la vasoconstriction : calmoduline et endothéline-1.
   → Ces effets sont en faveur d'une inhibition de l'angiogénèse et de la dilatation vasculaire, et montrent donc une activité anti-rougeur de GG.
- stimule l'expression de molécules impliquées dans le renforcement et l'élasticité des vaisseaux : αSMA (αSmooth Muscle Actin) et troponine 1.
- stimule l'expression de molécules de défense : Heme Oxygénase 1 (rôle protecteur en empêchant l'hème libre de participer à des réactions pro-oxydantes) et thioredoxine (réparation des dommages oxydatifs aux protéines).
   → Ces effets sont en faveur d'une activité de renforcement et de protection des parois vasculaires par GG.

**Tableau 12 : Screening d'activité en PCR array sur cellules endothéliales**

| | **Expression génique** (Quantité relative en % par rapport aux cellules contrôles) | | |
|---|---|---|---|
| | Cellules contrôles | **GG 0,01%** | **GG 0,02%** |
| **Facteurs de croissance pro-angiogéniques** | | | |
| PGF | 100 | **64** | **53** |
| PDGFB | 100 | **84** | **62** |
| VEGFR3 | 100 | **57** | **65** |

| **Molécules anti-angiogéniques** | | | |
|---|---|---|---|
| Thrombospondine-1 | 100 | **148** | **183** |
| Endostatine | 100 | **112** | **220** |

| **Vasoconstriction** | | | |
|---|---|---|---|
| Calmoduline | 100 | **120** | **165** |
| Endothéline 1 | 100 | **127** | **169** |

| **Renforcement des vaisseaux / Elasticité** | | | |
|---|---|---|---|
| αSMA | 100 | **127** | **183** |
| Troponine 1 | 100 | **177** | **184** |

| **Défense / Réponse au stress oxydatif** | | | |
|---|---|---|---|
| Heme Oxygenase 1 | 100 | **185** | **320** |
| Thioredoxine | 100 | **126** | **171** |

## Revendications

1. Composition comprenant à titre de principe actif un extrait de parties aériennes de *Gynandropsis gynandra,* **caractérisée en ce que** l'extrait comprend au moins 3 % en poids de polyphénols, exprimé en équivalent d'acide gallique par rapport au poids de l'extrait sec, lesdits polyphénols étant avantageusement des flavonoïdes, typiquement à une teneur d'au moins 1 % en poids de flavonoïdes, exprimé en équivalent de rutine par rapport au poids de l'extrait sec, et le cas échéant un excipient approprié, ledit extrait étant susceptible d'être obtenu par extraction solide-liquide des parties aériennes de *Gynandropsis gynandra* dans des mélanges binaires d'eau et de glycérol ou de glycol tel que le propanediol, ledit mélange binaire contenant l'eau dans des proportions comprises entre 10 et 50 % par rapport à l'autre solvant.

2. Composition selon la revendication 1, **caractérisée en ce que** la composition est destinée à être appliquée par voie topique externe sur la peau, les phanères et/ou les muqueuses, en particulier les peaux, les phanères et/ou les muqueuses sensibles ou agressées par l'environnement.

3. Composition selon les revendications 1 ou 2, **caractérisée en ce que** l'extrait est un extrait de feuilles de *Gynandropsis gynandra.*

4. Composition selon l'une quelconque des revendications précédentes, **caractérisée en ce que** les parties aériennes de *Gynandropsis gynandra* sont fraîches ou sèches, avantageusement sèches.

5. Composition selon l'une quelconque des revendications précédentes, comprenant en outre au moins un autre actif, en particulier choisi dans le groupe constitué par les émollients, les actifs hydratants, les kératorégulateurs, les kératolytiques, les agents cicatrisant et/ou restructurant de la barrière cutanée, les agents sébo-régulateurs, les agents anti-irritants et/ou anti-inflammatoires et/ou apaisants, les agents anti-oxydants, les agents anti-âge, les agents dépigmentants ou hypopigmentants, les agents pigmentants, les agents lipolytiques ou inhibiteurs de la lipogénèse ou encore les agents anti-cellulite ou amincissants, les filtres et écrans solaires minéraux ou organiques, les composés antifongiques, les conservateurs, les agents anti-bactériens, les pré et probiotiques, les antibiotiques, et les immunomodulateurs.

6. Composition selon la revendication 5, **caractérisée en ce que** l'autre actif est choisi parmi :
• les agents cicatrisant et/ou restructurant de la barrière cutanée, de préférence le panthénol,
• les agents sébo-régulateurs, de préférence choisis parmi les inhibiteurs de 5-alpha réductase, les dérivés de zinc, la spironolactone, et l'acide linoléique,
• les agents anti-inflammatoires et/ou anti-irritants et/ou apaisants, de préférence l'arabinogalactane,
• les filtres et écrans solaires minéraux ou organiques, de préférence les filtres et écrans solaires UVB et/ou UVA,
• les conservateurs de préférence choisis parmi le capryloyl glycine, le glycéryl caprylate, l'hexanediol, le sodium levulinate, les dérivés de zinc et de cuivre.

7. Composition selon l'une quelconque des revendications 1 à 4, comprenant en outre au moins un autre actif choisi dans le groupe constitué parmi :
• les huiles végétales, de préférence l'huile de soja, l'huile de colza, l'huile d'avocat, l'huile de lupin et avantageusement l'huile de lupin blanc doux, ou un mélange de ces huiles,
• les oléodistillats ou les concentrats d'huile végétale ou animale, de préférence de tournesol, d'avocat, de colza, de maïs et de palme et avantageusement concentrés en insaponifiables,
• les insaponifiables de végétaux ou d'huile végétale, de préférence les insaponifiables d'avocats, les insaponifiables de soja ou leurs mélanges, avantageusement des furanes d'avocat, et en particulier un mélange d'insaponifiables d'Avocat furaniques et d'insaponifiables de Soja dans un rapport respectif d'environ 1/3-2/3, les insaponifiables stéroliques, les phytostérols, les esters de stérols et les dérivés vitaminiques,
• les peptides ou complexes d'acides aminés végétaux, de préférence les peptides d'avocat, les peptides de lupin, les peptides de quinoa, les peptides de Maca, les peptides de soja fermentés ou non, les peptides de riz, les peptides de Schizandra, un extrait de graines d'*Acacia macrostachya* et un extrait de graines de *Vigna unguiculata,*
• les sucres de végétaux, de préférence les sucres d'avocat,
• le butyle avocadate,
• les extraits riches en polyphénols, de préférence les extraits d'avocat et les extraits de feuilles de Maca,
• le lupéol,
• un extrait total de lupin,
• les oxazolines, de préférence la 2-undécyl-4-hydroxyméthyl-4-méthyl-1,3-oxazoline, la 2-undécyl-4,4-diméthyl-1,3-oxazoline, la (E)-4,4-diméthyl-2-heptadéc-8-ényl-1,3-oxazoline, la 4-hydroxyméthyl-4-méthyl-2-heptadécyl-1,3-oxazoline, la (E)-4-hydroxyméthyl-4-méthyl-2-heptadéc-8-ényl-1,3-oxazoline, la 2-undécyl-4-éthyl-4-hydroxyméthyl-1,3-oxazoline et la 2-undécyl-4,4-diméthyl-1,3-oxazoline, et
• leurs mélanges.

8. Composition selon l'une quelconque des revendications précédentes, **caractérisée en ce qu'**il s'agit d'une composition cosmétique, dermatologique ou pharmaceutique.

9. Procédé de soin cosmétique de la peau et/ou des phanères et/ou des muqueuses, en vue d'améliorer leur état et/ou leur aspect, consistant à appliquer sur la peau et/ou phanères et/ou muqueuses des patients qui en ont besoin une composition selon l'une quelconque des revendications précédentes ou un extrait de parties aériennes de *Gynandropsis gynandra* tel que défini à l'une quelconque des revendications 1 à 4.

10. Procédé de soin cosmétique selon la revendication 9 pour les peaux, les phanères et/ou les muqueuses sensibles ou agressées par l'environnement, ou pour les peaux, les phanères et/ou les muqueuses présentant des rougeurs, typiquement avec des tiraillements ou des picotements quotidiens, ou pour réguler la production de sébum, ou encore les états pelliculaires.

11. Procédé de soin cosmétique selon les revendications 9 ou 10, **caractérisé en ce que** la composition ou l'extrait est un produit anti-âge chronologique ou photo-induit, ou encore un produit hydratant, amincissant et/ou anticellulite.

12. Composition selon l'une quelconque des revendications 1 à 8 pour son utilisation dans la prévention et/ou le traitement des troubles liés à l'hyperséborrhée, de préférence la dermite séborrhéique, ou encore de l'acné ou des peaux acnéiques, ou pour son utilisation en tant que produit cicatrisant, anti-allergique et/ou pro-pigmentant.

13. Composition selon l'une quelconque des revendications 1 à 8 pour son utilisation dans la prévention et/ou le traitement des troubles vasculaires.

14. Composition selon l'une quelconque des revendications 1 à 8 pour son utilisation dans la prévention et/ou le traitement des conditions suivantes : la couperose, les érythèmes cutanés, la rosacée, le prurit, la peau et/ou les muqueuses réactives, avec des rougeurs, en particulier dues à une dilatation des capillaires sous-cutanés.

## Patentansprüche

1. Zusammensetzung, die als Wirkstoff einen Extrakt aus den oberirdischen Pflanzenteilen von *Gynandropsis gynandra* enthält, **dadurch gekennzeichnet, dass** der Extrakt zumindest 3 Gew.-% Polyphenole, als Gallussäureäquivalent bezogen auf das Trockenextraktgewicht ausgedrückt, enthält, wobei die Polyphenole vorteilhaft Flavonoide sind, typischerweise mit einem Gehalt von zumindest 1 Gew.-% Flavonoiden, als Rutinäquivalent bezogen auf das Trockenextraktgewicht ausgedrückt, und gegebenenfalls einen geeigneten Hilfsstoff, wobei der Extrakt durch Fest-Flüssig-Extraktion der oberirdischen Pflanzenteile von *Gynandropsis gynandra* in binären Mischungen von Wasser und Glyzerol oder Glycol wie Propandiol gewonnen wird, wobei die binäre Mischung Wasser in Anteilen zwischen 10 und 50 % bezogen auf das andere Lösungsmittel enthält.

2. Zusammensetzung nach Anspruch 1, **dadurch gekennzeichnet, dass** die Zusammensetzung dazu bestimmt ist, auf topischem Wege auf die Haut, Hautanhangsgebilde und/oder Schleimhäute, insbesondere empfindliche oder umweltbelastete Haut, Hautanhangsgebilde und/oder Schleimhäute, aufgetragen zu werden.

3. Zusammensetzung nach Anspruch 1 oder 2, **dadurch gekennzeichnet, dass** der Extrakt ein Extrakt aus *Gynandropsis* gynandra-Blättern ist.

4. Zusammensetzung nach einem beliebigen der vorstehenden Ansprüche, **dadurch gekennzeichnet, dass** die oberirdischen Pflanzenteile von *Gynandropsis gynandra* frisch oder trocken, vorteilhaft trocken sind.

5. Zusammensetzung nach einem beliebigen der vorstehenden Ansprüche, die ferner zumindest einen weiteren Wirkstoff umfasst, der insbesondere aus der Gruppe bestehend aus Weichmachern, feuchtigkeitsspendenden Wirkstoffen, Keratoregulatoren, Keratolytika, Wirkstoffen zur Wundheilung und/oder Restrukturierung der Hautbarriere, Seboregulatoren, reizlindernden und/oder entzündungshemmenden und/oder beruhigenden Mitteln, Antioxydantien, Anti-Aging-Stoffen, Depigmentier- oder Hypopigmentierungsmitteln, Pigmentierungsmitteln, lipolytischen Mitteln oder Lipogenese-Hemmern oder auch Anti-Cellulite- oder Körperpflegemitteln, mineralischen oder organischen Sonnenschutzmitteln und Filtern, antimykotischen Mitteln, Konservierungsmitteln, antibakteriellen Mitteln, Prä- und Probiotika, Antibiotika und Immunmodulatoren ausgewählt ist.

6. Verfahren nach Anspruch 5, **dadurch gekennzeichnet, dass** der andere Wirkstoff ausgewählt ist unter:
• heilenden und/oder restrukturierenden Wirkstoffen der Hautbarriere, vorzugsweise Panthenol,
• seboregulatorischen Mitteln, vorzugsweise ausgewählt unter 5-Alpha-Reduktase-Hemmern, Zink-Derivaten, Spironolactonen und Linolsäure,
• entzündungshemmenden und/oder reizlindernden und/oder beruhigenden Mitteln, vorzugsweise Arabinogalactan,
• mineralischen oder organischen Filtern und Sonnenschutzmitteln, vorzugsweise UVB- und/oder UVA-Filtern und Sonnenschutzmitteln,
• Konservierungsmitteln, vorzugsweise ausgewählt aus Capryloylglycin, Glycerylcaprylat, Hexandiol, Natrium-Levulinat, Zink und Kupferderivaten.

7. Zusammensetzung nach einem beliebigen der vorstehenden Ansprüche 1 bis 4, die ferner zumindest einen weiteren Aktivstoff enthält, der ausgewählt ist aus der Gruppe bestehend aus Folgenden:
• Pflanzenöle, vorzugsweise Sojaöl, Rapsöl, Avocadoöl, Lupinenöl und vorteilhafterweise süßes weißes Lupinenöl oder eine Mischung dieser Öle,
• Oleodestillate oder Konzentrate von pflanzlichen oder tierischen Ölen, vorzugsweise Sonnenblumen, Avocado, Raps, Mais und Palmen und vorteilhafterweise in Unverseifbaren konzentriert,
• Unverseifbare aus Pflanzen oder Pflanzenöl, vorzugsweise Avocado Unverseifbare, Soja Unverseifbare oder Mischungen davon, vorzugsweise Avocadofurane, und insbesondere eine Mischung aus Avocadofuran Unverseifbaren und Soja Unverseifbaren im jeweiligen Verhältnis von etwa 1/3 - 2/3, Sterol Unverseifbare, Phytosterine, Sterolester und Vitamin-Derivate,
• pflanzliche Aminosäurepeptide oder -komplexe, vorzugsweise Avocadopeptide, Lupinpeptide, Quinoapeptide, Macapeptide, fermentierte oder unfermentierte Sojapeptide, Reispeptide, Schizandrapeptide, ein *d'Acacia macrostachya* Samenextrakt und ein *Vigna unguiculata* Samenextrakt,
• Pflanzenzucker, vorzugsweise Avocadozucker,
• Avocadobutyl,
• Extrakte, die reich an Polyphenolen sind, vorzugsweise Extrakte aus oberirdischen Pflanzenteilen de Gynandropsis gynandra und Extrakte aus Maca-Blättern,
• Lupeol,
• ein totaler Lupinenextrakt
• Oxazoline, vorzugsweise 2-undecyl-4-hydroxymethyl-4-methyl-1,3-oxazoline, la 2-undecyl-4,4-dimethyl-1,3-oxazoline, la (E)-4,4-dimethyl-2-heptadec-8-enyl-1,3-oxazoline, la 4- hydroxymethyl-4-methyl-2-heptadecyl-1,3-oxazoline, (E)-4- hydroxymethyl-4-methyl-2-heptadec-8-enyl-1,3-oxazoline, 2- undecyl-4-ethyl-4-hydroxymethyl-1,3-oxazoline und 2-undecyl-4,4-dimethyl-1,3-oxazoline, und
• Mischungen davon.

8. Zusammensetzung nach einem beliebigen der vorstehenden Ansprüche, **dadurch gekennzeichnet, dass** es sich um eine kosmetische, dermatologische oder pharmazeutische Zusammensetzung handelt.

9. Kosmetisches Behandlungsverfahren der Haut und/oder der Hautanhangsgebilde und/oder der Schleimhäute, um ihren Zustand und/oder ihr Aussehen zu verbessern, wobei eine Zusammensetzung nach einem beliebigen der vorstehenden Ansprüche oder ein Extrakt aus den oberirdischen Pflanzenteilen von *Gynandropsis gynandra,* wie in einem beliebigen der vorstehenden Ansprüche 1 bis 4 definiert, auf die Haut und/oder die Hautanhangsgebilde und/oder die Schleimhäute von Patienten, die sie benötigen, aufgetragen wird.

10. Kosmetisches Behandlungsverfahren nach Anspruch 9 für empfindliche oder umweltbelastete Haut, Hautanhangsgebilde und/oder Schleimhäute, oder für gerötete Haut, Hautanhangsgebilde und/oder Schleimhäute, typischerweise mit täglichem Spannungsgefühl oder Kribbeln oder zur Regulierung der Talgproduktion oder auch Schuppenbildung.

11. Kosmetisches Behandlungsverfahren nach Anspruch 9 oder 10, **dadurch gekennzeichnet, dass** die Zusammensetzung oder der Extrakt ein chronologisches oder photoinduziertes Anti-Ageing-Produkt oder ein feuchtigkeitsspendendes, körperpflegendes und/oder Anti-Cellulite-Produkt ist.

12. Zusammensetzung nach einem beliebigen der vorstehenden Ansprüche 1 bis 8 zur Verwendung bei der Vorbeugung und/oder Behandlung von Erkrankungen im Zusammenhang mit Hyperseborrhoe, vorzugsweise seborrhoischer Dermatitis, Akne oder zu Akne neigender Haut, oder zur Verwendung als heilendes, antiallergisches und/oder pro-pigmentierendes Produkt.

13. Zusammensetzung nach einem beliebigen der vorstehenden Ansprüche 1 bis 8 zur Verwendung bei der Vorbeugung und/oder Behandlung von Gefäßerkrankungen.

14. Zusammensetzung nach einem beliebigen der vorstehenden Ansprüche 1 bis 8 zur Verwendung bei der Vorbeugung und/oder Behandlung folgender Erkrankungen: Couperose, Hauterythem, Rosacea, Juckreiz, reaktive Haut und/oder Schleimhäute, mit Rötungen, insbesondere durch Erweiterung der subkutanen Kapillaren.

## Claims

1. A composition including as active ingredient an extract of the above-ground parts of *Gynandropsis gynandra,* **characterized in that** the extract includes at least 3% polyphenols by weight, expressed in gallic acid equivalents in relation to the weight of the dry extract, said polyphenols being advantageously flavonoids, typically at a concentration of at least 1% flavonoids by weight, expressed in rutin equivalents in relation to the weight of the dry extract, and if need be a suitable carrier, said extract being obtainable by solid-liquid extraction of the above-ground parts of *Gynandropsis gynandra* in binary mixtures of water and glycerol or a glycol such as propanediol, said binary mixture containing water in proportions between 10 and 50% in relation to the other solvent.

2. The composition of claim 1, **characterized in that** the composition is intended to be applied by topical external route to the skin, keratinous appendages and/or mucous membranes, in particular skin, keratinous appendages and/or mucous membranes that are sensitive or damaged by the environment.

3. The composition of claim 1 or 2, **characterized in that** the extract is an extract of *Gynandropsis gynandra* leaves.

4. The composition of any one of the preceding claims, **characterized in that** the above-ground parts of *Gynandropsis gynandra* are fresh or dried, advantageously dried.

5. The composition of any one of the preceding claims, further including at least one other active agent, in particular selected from the group consisting of emollients, moisturizing active agents, keratoregulators, keratolytics, healing and/or restructuring agents of the cutaneous barrier, sebum-regulating agents, anti-irritation and/or anti-inflammatory and/or soothing agents, antioxidant agents, anti-aging agents, depigmenting or hypopigmenting agents, pigmenting agents, lipolytic or lipogenesis inhibitor agents or anti-cellulitis or slimming agents, organic or mineral sun screens and filters, antifungal compounds, preservatives, antibacterial agents, prebiotics and probiotics, antibiotics, and immunomodulators.

6. The composition of claim 5, **characterized in that** the other active agent is selected from:
• healing and/or restructuring agents of the cutaneous barrier, preferably panthenol,
• sebum-regulating agents, preferably selected from 5-alpha reductase inhibitors, zinc derivatives, spironolactone and linoleic acid,
• anti-inflammatory and/or anti-irritation and/or soothing agents, preferably arabinogalactan,
• mineral or organic sun filters and screens, preferably UVB and/or UVA sun filters and screens,
• preservatives preferably selected from capryloyl glycine, glyceryl caprylate, hexanediol, sodium levulinate, and copper and zinc derivatives.

7. The composition of any one of claims 1 to 4, further including at least one other active agent selected from the group consisting of:
• plant oils, preferably soy oil, rapeseed oil, avocado oil, lupin oil and advantageously sweet white lupin oil, or a mixture of these oils,
• oleodistillates or concentrates of animal or plant oil, preferably sunflower, avocado, rapeseed, corn and palm oil and advantageously concentrated in unsaponifiables,
• unsaponifiables of plants or plant oil, preferably avocado unsaponifiables, soy unsaponifiables or mixtures thereof, advantageously avocado furans, and in particular a mixture of furanic unsaponifiables of avocado and unsaponifiables of soy in a respective ratio of about 1/3-2/3, sterolic unsaponifiables, phytosterols, esters of sterols and vitamin derivatives,
• peptides or complexes of plant amino acids, preferably avocado peptides, lupin peptides, quinoa peptides, maca peptides, fermented or non-fermented soy peptides, rice peptides, *Schisandra* peptides, extract of *Acacia macrostachya* seeds and extract of *Vigna unguiculata* seeds,
• plant sugars, preferably avocado sugars,
• butyl avocadate,
• polyphenol-rich extracts, preferably extracts of avocado and extracts of maca leaves,
• lupeol,
• a total extract of lupin,
• oxazolines, preferably 2-undecyl-4-hydroxymethyl-4-methyl-1,3-oxazoline, 2-undecyl-4,4-dimethyl-1,3-oxazoline, (E)-4,4-dimethyl-2-heptadec-8-enyl-1,3-oxazoline, 4-hydroxymethyl-4-methyl-2-heptadecyl-1,3-oxazoline, (E)-4-hydroxymethyl-4-methyl-2-heptadec-8-enyl-1,3-oxazoline, 2-undecyl-4-ethyl-4-hydroxymethyl-1,3-oxazoline and 2-undecyl-4,4-dimethyl-1,3-oxazoline, and
• mixtures thereof.

8. The composition of any one of the preceding claims, **characterized in that** it is a cosmetic, dermatological or pharmaceutical composition.

9. A cosmetic care method for the skin and/or keratinous appendages and/or mucous membranes, with a view to improving the condition and/or appearance thereof, consisting in applying to the skin and/or keratinous appendages and/or mucous membranes of patients in need a composition according to any one of the preceding claims or an extract of the above-ground parts of *Gynandropsis gynandra* as defined in any one of claims 1 to 4.

10. The cosmetic care method of claim 9 for skin, keratinous appendages and/or mucous membranes that are sensitive or damaged by the environment, or for skin, keratinous appendages and/or mucous membranes presenting redness, typically with daily tugging or tingling, or for regulating sebum production, or dandruff.

11. The cosmetic care method of claim 9 or 10, **characterized in that** the composition or extract is a chronological or photo-induced anti-aging product, or a moisturizing, slimming and/or anti-cellulitis product.

12. The composition of any one of claims 1 to 8 to be used in the prevention and/or treatment of disorders related to hyperseborrhea, preferably seborrheic dermatitis, or acne or acneic skin, or to be used as a healing, anti-allergy and/or pro-pigmenting product.

13. The composition of any one of claims 1 to 8 to be used in the prevention and/or treatment of vascular disorders.

14. The composition of any one of claims 1 to 8 to be used in the prevention and/or treatment of the following conditions: couperosis, cutaneous erythema, rosacea, pruritus, reactive skin and/or mucous membranes, with redness, in particular due to dilatation of the subcutaneous capillaries.
